# EUROPÄISCHE PATENTANMELDUNG

(11) **EP 4 744 845 A1**
(43) Veröffentlichungstag der Anmeldung: **20.05.2026**
(21) Anmeldenummer: 25209652.4
(22) Anmeldetag: 20.10.2025
(51) Int. Cl.: B25J 19/00, A61B 90/50, A61B 18/20, A61N 5/067

(54) **DREHGELENK FÜR EINEN SPIEGELGELENKARM, SPIEGELGELENKARM, SOWIE LASERSYSTEM MIT EINEM SOLCHEN SPIEGELGELENKARM**

(30) Priorität: 19.11.2024 DE 102024133792
(71) Anmelder: Karl Leibinger Asset Management GmbH & Co. KG, 78570 Mühlheim an der Donau (DE)
(72) Erfinder: Heinz, Oliver, 79104 Freiburg (DE); Riesle, Klaus, 79312 Emmendingen (DE); Kiefer, Philip, 79424 Auggen (DE); Martin, Lukas, 79100 Freiburg (DE)
(74) Vertreter: Schwamberger, Martin

(57) **Zusammenfassung**

Drehgelenk (40) für einen Spiegelgelenkarm (30), mit zwei Gelenkelementen (42, 43), welche um eine Drehachse drehbar miteinander gekoppelt und innerhalb eines Arbeitsbereichs (A) gegeneinander verschwenkbar sind, wobei das Drehgelenk (40) eine Drehfederanordnung (50) umfasst, die im Arbeitsbereich (A) mit den Gelenkelementen (42, 43) in Eingriff steht und ein Drehmoment zwischen diesen überträgt, um im Arbeitsbereich (A) zumindest einen Teil des Eigengewichts eines mit einem der Gelenkelemente (42, 43) verbundenen Elements (30) zu kompensieren, wobei das Drehgelenk (40) von dem Arbeitsbereich (A) in eine Parkposition (P) verschwenkbar ist, wobei das Drehgelenk (40) dazu eingerichtet ist, die Drehfederanordnung (50) beim Verlassen des Arbeitsbereiches (A) in Richtung auf die Parkposition (P) mit mindestens einem der Gelenkelemente (42, 43) selbsttätig außer Eingriff zu bringen, so dass die Drehfederanordnung (50) einer Schwenkbewegung in die Parkposition (P) kein Drehmoment entgegensetzt, sowie Spiegelgelenkarm (30) für ein Lasersystem (10) mit einem solchen Drehgelenk (40), sowie Lasersystem (10) mit einem solchen Spiegelgelenkarm (30).

## Beschreibung

Die vorliegende Erfindung betrifft ein Drehgelenk für einen Spiegelgelenkarm eines Lasersystem zur Übertragung von Laserenergie von einer Laserquelle zu einem Applikator, insbesondere für den Spiegelgelenkarm eines medizinischen, insbesondere chirurgischen Lasersystems. Die Erfindung betrifft ferner einen Spiegelgelenkarm, sowie ein Lasersystem mit einem solchen Spiegelgelenkarm.

Durch Verschwenken eines Gelenkarms über ein Drehgelenk kann ein Applikator, welcher am Endglied des Gelenkarms angeordnet ist, bewegt werden. Bei solchen Systemen wird das erste Gelenkarm-Segment üblicherweise in einer aufrechten Arbeitsposition (z.B. ca. 15-90° aus der Vertikalen) eingesetzt, welche durch Verschwenken des Drehgelenks, an welchem das Gelenkarm-Segment angeordnet ist, verändert werden kann. Zum Verstauen wird der Arm in eine Parkposition gebracht, wozu er entgegen der Arbeitsposition nach unten geklappt wird.

Im Arbeitsbereich sollte sich der Arm zumindest weitgehend selbst halten, also sein Eigengewicht kompensieren, um ein ausbalanciertes Führen des Applikators zu gewährleisten.

Üblicherweise werden Spiegelgelenkarme im Arbeitsbereich mit Drehfedern oder einem Gegengewicht im Gleichgewicht gehalten. So lehrt beispielsweise die WO 2012/086055 A1 einen Manipulator für ein Lasergerät, mit einem Stützmechanismus zum Abstützen eines Gelenkarms. Eine Drehfeder des Stützmechanismus dient dabei zur Erzeugung eines Ausgleichsmoments.

Manche Arme beinhalten zusätzlich eine einstellbare Bremse, um den Gewichtsbereich, in dem der Arm in Balance ist, zu erweitern. Zum Übergang von der Arbeitsposition in die Parkposition bzw. von der Parkposition zur Arbeitsposition muss bei bekannten Gelenkarmen ein Bedienelement betätigt werden, um einen mechanischen Anschlag zu lösen. Je nach Ausführung ist zum Betätigen des Bedienelements ein vorheriges Entlasten des Arms notwendig, da ansonsten die Federvorspannung ein Lösen der Arretierung verhindert.

Aufgabe der vorliegenden Erfindung ist es daher, ein verbessertes Drehgelenk für einen Spiegelgelenkarm zur Verfügung zu stellen, um die Bedienung eines mit einem solchen Drehgelenk ausgestatteten Arm zu vereinfachen und intuitiver zu gestalten.

Die Aufgabe wird erfindungsgemäß durch ein Drehgelenk für einen Spiegelgelenkarm nach Anspruch 1 gelöst. Bevorzugte Ausgestaltungen des erfindungsgemäßen Drehgelenks sind Gegenstand der Unteransprüche. Weitere vorteilhafte Ausführungen ergeben sich aus der Beschreibung sowie aus den Figuren.

Die vorliegende Erfindung umfasst ein Drehgelenk für einen Spiegelgelenkarm eines Lasersystem zur Übertragung von Laserenergie von einer Laserquelle zu einem Applikator, mit einem ersten und einem zweiten Gelenkelement, welche um eine Drehachse drehbar miteinander gekoppelt sind. Hierbei sind das erste und das zweite Gelenkelement innerhalb eines Arbeitsbereichs gegeneinander verschwenkbar, wobei das Drehgelenk eine Drehfederanordnung umfasst, die im Arbeitsbereich mit dem ersten und dem zweiten Gelenkelement in Eingriff steht und ein Drehmoment zwischen diesen überträgt, um im Arbeitsbereich zumindest einen Teil des Eigengewichts eines mit einem der Gelenkelemente verbundenen Elements zu kompensieren. Weiterhin ist das Drehgelenk von dem Arbeitsbereich in eine Parkposition und zurück verschwenkbar. Erfindungsgemäß ist das Drehgelenk dazu eingerichtet, dass die Drehfederanordnung beim Verlassen des Arbeitsbereiches in Richtung auf die Parkposition mit mindestens einem der Gelenkelemente selbsttätig außer Eingriff kommt, so dass die Drehfederanordnung einer Schwenkbewegung in die Parkposition kein Drehmoment entgegensetzt. Erfindungsgemäß ist daher ein widerstandfreies, bzw. widerstandsarmes Überführen in die Parkposition möglich. Weiterhin ist kein Bedienelement notwendig, um die Drehfederanordnung außer Eingriff zu nehmen. Die Bedienung ist daher einfacher und intuitiver gestaltet und mögliche Bedienfehler werden minimiert.

Gemäß einer möglichen Ausgestaltung weist das Drehgelenk einen Anschlag auf, welcher den Arbeitsbereich auf der der Parkposition abgewandten Seite begrenzt und so ein Überdrehen der Drehfederanordnung verhindert. Auch hierdurch wird eine einfachere Bedienung ermöglicht, da eindeutig wird, in welche Drehrichtung in die Parkposition eingeschwenkt werden muss. Zudem wird eine Beschädigung des Drehgelenks vermieden.

Gemäß einer möglichen Ausgestaltung weist das Drehgelenk mindestens ein Rastelement auf, welches ein Rastmoment erzeugt, das beim Verlassen des Arbeitsbereiches und/oder beim Verlassen der Parkposition überwunden werden muss. Das Rastmoment erzeugt zusätzliche Sicherheit gegen unbeabsichtigtes Aus- / Einklappen.

Gemäß einer möglichen Ausgestaltung wird das Rastelement durch zumindest ein Federblech gebildet, welches bevorzugt mit einem Stiftelement zusammenwirkt, insbesondere ein gebogenes Federblech. Besonders bevorzugt werden mehrere dünne Federbleche zu einem Federblechpaket zusammengefasst, um das gewünschte Rastmoment zu erzeugen.

Gemäß einer möglichen Ausgestaltung umfasst das Drehgelenk eine Bremsanordnung zum Abbremsen einer Verdrehung zwischen dem ersten und dem zweiten Gelenkelement, welche zumindest im Schwenkbereich zwischen dem Arbeitsbereich und der Parkposition wirksam oder betätigbar ist. Die Bremse dämpft die Bewegung des Drehgelenks, so dass zu schnelle Bewegungen und mögliche Schäden verhindert werden. Die Bremsanordnung kann beispielsweise durch einen O-Ring im Kraftfluss zwischen den beiden Gelenkelementen realisiert sein, sodass die Bremswirkung durch Reibung an der O-Ring Oberfläche erzielt wird. Es sind jedoch auch andere Ausführungen zur Realisierung einer solchen Bremsanordnung denkbar.

Gemäß einer möglichen Ausgestaltung ist die Bremsanordnung auch im Arbeitsbereich wirksam und dämpft auch dort eine Bewegung des Drehgelenks.

Gemäß einer weiteren möglichen Ausgestaltung der ist die Bremsanordnung so ausgestaltet, dass sie unabhängig von der Drehstellung des Drehgelenks wirksam ist.

Gemäß einer möglichen Ausgestaltung weist die Drehfederanordnung mindestens zwei Drehfedern auf. Gemäß einer möglichen Ausgestaltung sind die zwei Drehfedern gegenüberliegend angeordnet und/oder kommen unabhängig voneinander mit dem mindestens einen Gelenkelement in Eingriff und außer Eingriff. Hierdurch können die auf die Gelenkelemente wirkenden Kräfte ausgeglichen werden.

Gemäß einer möglichen Ausgestaltung bleibt die Drehfederanordnung in einem Schwenkbereich zwischen der Parkposition und dem Arbeitsbereich außer Eingriff mit dem mindestens einen Gelenkelement. Hierdurch bleibt die Schwenkbewegung über den Schwenkbereich zumindest weitgehend widerstandsfrei, bzw. widerstandsarm.

Gemäß einer möglichen Ausgestaltung kommt die Drehfederanordnung bei einer Bewegung aus der Parkposition zum Arbeitsbereich beim Eintritt in den Arbeitsbereich selbsttätig mit mindestens einem der Gelenkelemente in Eingriff. Hierdurch wird wiederum die Bedienbarkeit verbessert.

Gemäß einer möglichen Ausgestaltung weist die Drehfederanordnung im Schwenkbereich zwischen der Parkposition und dem Arbeitsbereich eine Vorspannung auf, welche beim Eintritt in den Arbeitsbereich einer weiteren Drehbewegung entgegenwirkt. Hierdurch wird dem Gewicht des mit einem der Gelenkelemente verbundenen Elements unmittelbar mit Eintritt in den Arbeitsbereich ein ausreichend großes Gegenmoment entgegengesetzt, um dieses auszubalancieren.

Gemäß einer möglichen Ausgestaltung weist die Drehfederanordnung mindestens ein Drehmomentübertragungselement auf, welches in dem Arbeitsbereich in Eingriff mit mindestens einem Gegenelement ist, auf welches es ein Drehmoment überträgt, wobei das Drehmomentübertragungselement und das Gegenelement beim Verlassen des Arbeitsbereiches selbsttätig außer Eingriff kommen.

Gemäß einer möglichen Ausgestaltung handelt es sich bei dem Drehmomentübertragungselement und/oder dem Gegenelement um einen Anschlag. Dies erlaubt eine besonders einfache konstruktive Umsetzung des automatischen Eingriffs zwischen Drehmomentübertragungselement und Gegenelement.

Gemäß einer möglichen Ausgestaltung kommen das Drehmomentübertragungselement und/oder das Gegenelement bei einer Schwenkbewegung von der Parkposition in Richtung auf den Arbeitsbereich beim Eintritt in den Arbeitsbereich miteinander in Anlage, so dass die Drehfederanordnung einer weiteren Schwenkbewegung ein Drehmoment entgegensetzt.

Gemäß einer möglichen Ausgestaltung kommen das Drehmomentübertragungselement und das Gegenelement bei einer Schwenkbewegung in die Gegenrichtung bei Verlassen des Arbeitsbereichs außer Kontakt.

Gemäß einer möglichen Ausgestaltung steht das Drehmomentübertragungselement im Arbeitsbereich in Eingriff mit dem Gegenelement und in einem Schwenkbereich zwischen dem Ende des Arbeitsbereiches und der Parkposition außer Eingriff mit dem Gegenelement.

Gemäß einer möglichen Ausgestaltung handelt es sich bei dem Drehmomentübertragungselement oder bei dem Gegenelement um ein Stiftelement und bei dem jeweils anderen Element um das Ende einer Nut, in welcher sich das Stiftelement bewegt. Dies erlaubt eine einfache konstruktive Umsetzung.

Gemäß einer möglichen Ausgestaltung umfasst die Drehfederanordnung mindestens eine Drehfeder, deren erstes Ende mit dem ersten Gelenkelement gekoppelt und insbesondere drehfest verbunden ist, und deren zweites Ende mit einem Drehelement gekoppelt und insbesondere drehfest verbunden ist, welches gegenüber dem ersten und dem zweiten Gelenkelement drehbar angeordnet ist. Das Drehmomentübertragungselement ist an dem Drehelement angeordnet und wirkt mit dem Gegenelement zusammen, welches mit dem zweiten Gelenkelement gekoppelt ist.

Gemäß einer möglichen Ausgestaltung handelt es sich bei dem Drehelement bevorzugt um ein Ringelement, welches drehbar um das erste oder zweite Gelenkelement und/oder drehbar zwischen dem ersten und dem zweiten Gelenkelement angeordnet ist.

Gemäß einer möglichen Ausgestaltung kommt das Drehelement an mindestens einem Ende des Arbeitsbereiches in Eingriff mit dem ersten Gelenkelement, so dass eine weitere Relativdrehung zwischen dem Drehelement und dem ersten Gelenkelement verhindert wird. Hierdurch wird entweder der Arbeitsbereich begrenzt und ein Überdrehen verhindert oder eine Vorspannung der Drehfederanordnung erzeugt.

Gemäß einer möglichen Ausgestaltung ist an dem Drehelement ein zweites Drehmomentübertragungselement angeordnet, welches an dem mindestens einem Ende des Arbeitsbereiches mit mindestens einem zweiten Gegenelement, welches an dem ersten Gelenkelement angeordnet ist, in Eingriff kommt.

Gemäß einer möglichen Ausgestaltung handelt es sich bei dem mindestens einen zweiten Gegenelement und/oder zweiten Drehmomentübertragungselement um einen Anschlag.

Gemäß einer weiteren möglichen Ausgestaltung werden das zweite Drehmomentübertragungselement oder das zweite Gegenelement durch ein Stiftelement und das jeweils andere Element durch das Ende einer Nut, in welcher sich das Stiftelement bewegt, gebildet.

Gemäß einer möglichen Ausgestaltung umfasst das Drehgelenk einen Strahlführungsbereich, welcher entlang der Drehachse durch das Drehgelenk hindurchführt, so dass ein Laserstrahl koaxial zur Drehachse durch das Drehgelenk hindurchführbar ist. Der Strahlführungsbereich weist hierbei bevorzugt im Querschnitt über seine gesamte axiale Erstreckung eine lichte Weite von mindestens 5 mm, bevorzugt von mindestens 10 mm, weiter bevorzugt von mindestens 15 mm auf.

Insbesondere kann es sich bei dem Strahlführungsbereich um ein Strahlführungsrohr handeln, welches durch das Drehgelenk hindurchführt und die Drehachse umgibt, insbesondere koaxial umgibt.

Gemäß einer möglichen Ausgestaltung handelt es sich bei den beiden Gelenkelementen um eine Welle und ein die Welle umgebendes Gehäuse, wobei die Welle drehbar im Gehäuse gelagert ist.

Gemäß einer möglichen Ausgestaltung umgibt eine Drehfeder der Drehfederanordnung die Welle und ist im Bereich zwischen der Welle und dem Gehäuse angeordnet.

Die oben beschriebenen Nuten sind hierbei bevorzugt an der Welle bzw. am Gehäuse vorgesehen.

Bevorzugt ist die Drehfeder der Drehfederanordnung mit der Welle gekoppelt, insbesondere drehfest verbunden.

Gemäß einer möglichen Ausgestaltung handelt es sich bei der Welle um eine Hohlwelle, innerhalb welcher sich ein Strahlführungsbereich axial durch das Drehgelenk hindurch erstreckt.

Gemäß einer möglichen Ausgestaltung ist innerhalb der Welle ein Strahlführungsrohr drehbar gelagert, welches koaxial durch das Drehgelenk hindurch führt.

Gemäß einer möglichen Ausgestaltung ist an dem Drehgelenk ein Stützarm angeordnet oder anordenbar, welcher ein Strahlführungsrohr des Gelenkarm-Segments stützt. Hierdurch werden die Stützkräfte der Federanordnung erst im Bereich des Strahlführungsrohres eingeleitet.

Die vorliegende Erfindung umfasst weiterhin einen Spiegelgelenkarm für ein Lasersystem zur Übertragung von Laserenergie von einer Laserquelle zu einem Applikator, mit einer Basis, einem Gelenkarm-Segment und einem Drehgelenk, wie es oben beschrieben wurde. Eines der Gelenkelemente des Drehgelenks ist dabei mit dem Gelenkarm-Segment verbunden oder verbindbar. Das andere der Gelenkelemente des Drehgelenks ist mit der Basis verbunden oder verbindbar, sodass durch Verschwenken der beiden Gelenkelemente eine Arbeitsposition des Gelenkarm-Segments relativ zur Basis veränderbar ist. Die Drehfederanordnung des Drehgelenks ist dazu eingerichtet, zumindest einen Teil des Eigengewichts des Gelenkarm-Segments zu kompensieren.

Gemäß einer möglichen Ausgestaltung weist der Spiegelgelenkarm eine Mehrzahl von gegeneinander drehbaren Gelenkarm-Segmenten auf.

Gemäß einer möglichen Ausgestaltung ist die Drehachse des Drehgelenks mit der Federanordnung horizontal angeordnet.

Gemäß einer möglichen Ausgestaltung ist das Drehgelenk mit der Federanordnung zwischen einer um eine vertikal ausgerichtete Achse drehbaren Basis und einem ersten Gelenkarm-Segment des Spiegelgelenkarms angeordnet.

Gemäß einer möglichen Ausgestaltung liegen sich der Arbeitsbereich und zumindest ein Teil des Schwenkbereichs zwischen dem Arbeitsbereich und der Parkposition bezüglich der Vertikalen gegenüber, sodass das Gelenkarm-Segment nach dem Verlassen des Arbeitsbereichs auf dem Weg zur Parkposition durch eine obere vertikale Stellung schwenkbar ist.

Gemäß einer möglichen Ausgestaltung weist der Beginn des Arbeitsbereichs einen Winkelabstand zu einer oberen vertikalen Stellung von mindestens 5°, insbesondere von mindestens 10° und/oder von höchstens 25°, bevorzugt höchstens 20° auf.

Gemäß einer möglichen Ausgestaltung steht das Gelenkarm-Segment in der Parkposition in einem Winkel von höchstens 10° zu einer unteren vertikalen Stellung.

Gemäß einer möglichen Ausgestaltung weist der Arbeitsbereich eine Größe von weniger als 90° auf.

Gemäß einer möglichen Ausgestaltung weist der Schwenkweg von dem Arbeitsbereich zur Parkposition eine Größe von mindestens 180° auf.

Der Spiegelgelenkarm weist Spiegel auf, welche den Laserstrahl entlang der Segmente des Spiegelgelenkarms führen. Weiterhin können die Segmente jeweils Strahlführungsrohe aufweisen, in welchen der Laserstrahl verläuft.

Die vorliegende Erfindung umfasst weiterhin ein Lasersystem mit einer Laserquelle und/oder einem Applikator und einem Spiegelgelenkarm, wie er oben beschrieben wurde. Bei dem Lasersystem kann es sich insbesondere um ein medizinisches Lasersystem, insbesondere ein chirurgisches Lasersystem handeln.

Ausführungsbeispiele der Erfindung sind anhand der Figuren detailliert beschrieben. Es zeigen:
- Fig. 1A und 1B: ein Ausführungsbeispiel eines Lasersystems mit einem Spiegelgelenkarm in der Arbeitsposition und in der Parkposition,
- Fig. 2: ein Ausführungsbeispiel eines Spiegelgelenkarms mit einem Drehgelenk,
- Fig. 3: eine Prinzipdarstellung der Positionen und Schwenkbereiche des Spiegelgelenkarms und Drehgelenks,
- Fig. 4: eine Schnittansicht entlang der Drehachse durch ein Ausführungsbeispiel des Drehgelenks,
- Fig. 5: eine Seitenansicht eines inneren Teils des Drehgelenks,
- Fig. 6: eine perspektivische Schnittansicht entlang der Drehachse des inneren Teils des Drehgelenks,
- Fig. 7: eine Schnittansicht durch das Drehgelenk senkrecht zur Drehachse,
- Fig. 8A bis 8D: Schnittansichten senkrecht zur Drehachse des Drehgelenks für eine Neutralstellung, eine Endstellung des Arbeitsbereichs, eine Stellung zwischen dem Arbeitsbereich und der Parkposition und für die Parkposition,
- Fig. 9: eine perspektivische Detailansicht des Drehgelenks mit einem Rastelement, und
- Fig. 10A, 10B: zwei Seitenansichten des inneren Teils des Drehgelenks mit einem Rastelement.

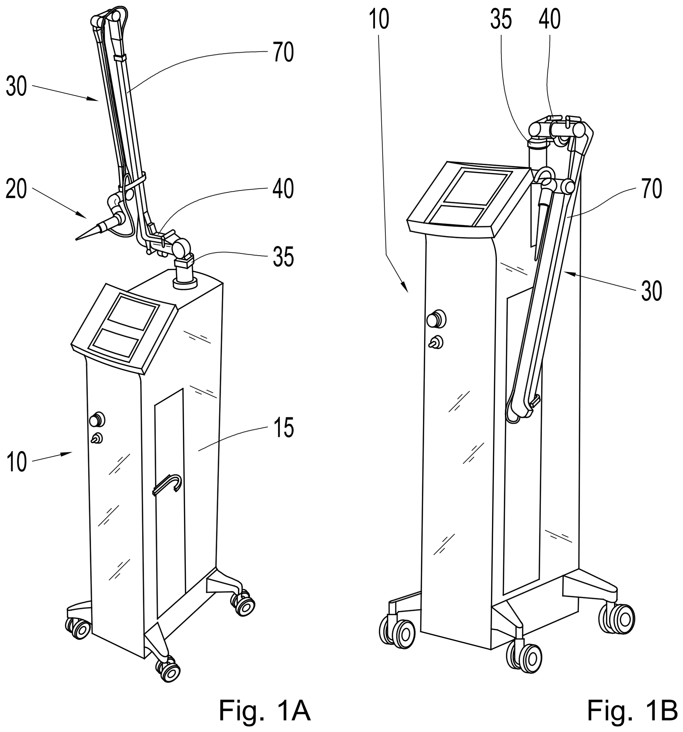

Fig. 1A zeigt ein Ausführungsbeispiel eines Lasersystems 10 mit einer Laserquelle 15, einem Spiegelgelenkarm 30 und einem Applikator 20. Insbesondere handelt es sich bei dem Lasersystem um ein medizinisches Lasersystem, insbesondere ein chirurgisches Lasersystem.

Die Laserquelle 15 umfasst im Ausführungsbeispiel ein Gehäuse, an welchem der Spiegelgelenkarm 30 montiert ist, insbesondere auf einer Oberseite des Gehäuses. Im Ausführungsbeispiel handelt es sich bei dem Lasersystem 10 um eine mobile Einheit, bei welcher die Laserquelle auf Rollen montiert ist, über welche das Lasersystem 10 bewegt werden kann. Das Lasersystem 10 weist im Ausführungsbeispiel ein Bedienfeld auf, über welche Parameter der Laserquelle 15 einstellbar sind.

Der Spiegelgelenkarm 30 dient der Übertragung der Laserenergie bzw. eines Laserstrahls von der Laserquelle 15 zum Applikator 20, welcher an einem Endsegment 120 des Spiegelgelenkarms 30 montiert ist.

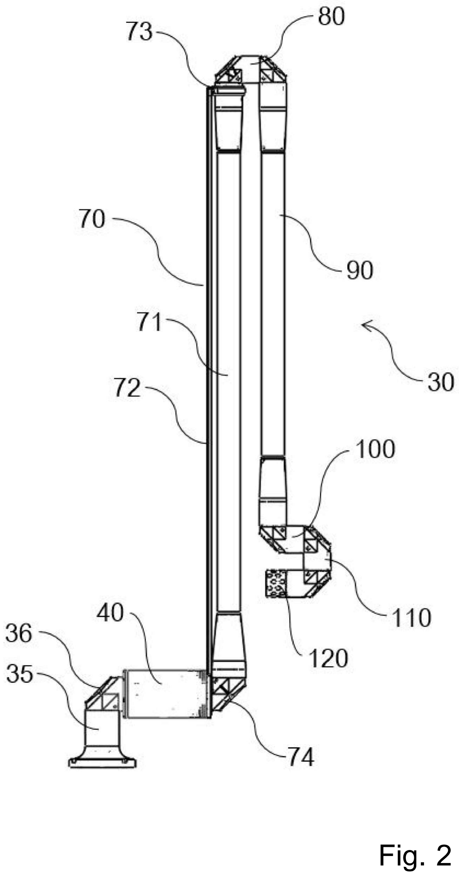

Der Spiegelgelenkarm 30 weist im Ausführungsbeispiel, wie in Fig. 2 näher gezeigt, mehrere Gelenkarm-Segmente 70, 90, 120 auf, welche über Drehgelenke 40, 80, 100, 110 gelenkig miteinander in Verbindung stehen, so dass das Endsegment 120 und der daran angeordnete Applikator 20 innerhalb eines Arbeitsbereiches frei bewegt und/oder ausgerichtet werden können. Die Gelenkarm-Segmente 35, 70, 90, 120 weisen jeweils Rohrelemente auf, in welchen der Laserstrahl geführt ist. Dort, wo die Rohrelemente (rechtwinklig) aufeinander treffen, sind Spiegelelemente 36, 74 vorgesehen, welche den Laserstahl so ablenken, dass er durch die Rohrelemente hindurch verläuft.

Der Spiegelgelenkarm 30 weist im Ausführungsbeispiel eine Basis 35 auf, mit welcher er an der Laserquelle 15 montiert ist und welche um eine vertikal ausgerichtete Drehachse drehbar ist.

An der Basis 35 ist ein Drehgelenk 40 angeordnet, über welches das Gelenkarm-Segment 70 um eine horizontal ausgerichtete Drehachse verschwenkbar ist.

Im Ausführungsbeispiel ist am distalen Ende des Gelenkarm-Segments 70 über das Drehgelenk 80 ein weiteres Gelenkarm-Segment 90 angeordnet. Dessen distales Ende trägt über Drehgelenke 100 und 110 das Endsegment 120, an welchem der Applikator 20 lösbar befestigt ist.

In Fig. 1A ist der Spiegelgelenkarm 30 in einer aufrechten Arbeitsposition gezeigt. Zum Verstauen kann der Spiegelgelenkarm 30 wie in Fig. 1B gezeigt in eine Parkposition gebracht werden, in welcher er nach unten geklappt seitlich an der Laserquelle 15 nach unten angeordnet ist.

Die jeweiligen Positionen des Gelenkarms 30 werden hierbei durch die Schwenk-Stellung des Gelenkarm-Segments 70 um die Drehachse des Drehgelenks 40 definiert.

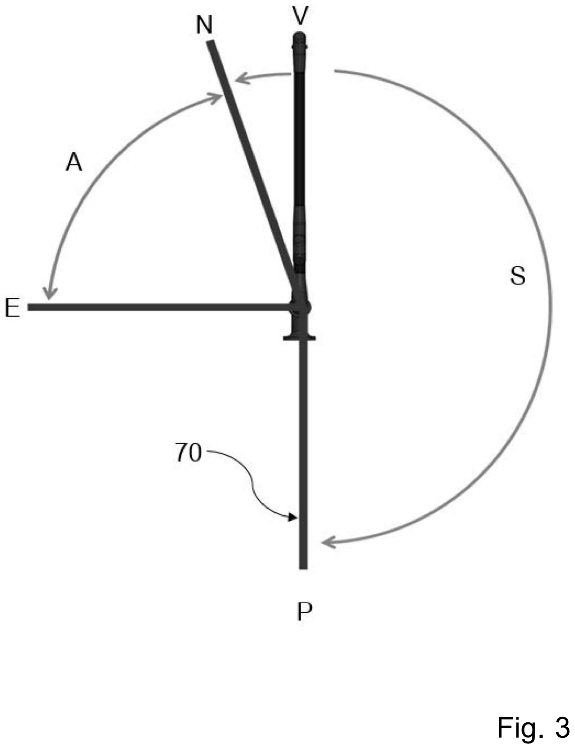

Wie in Fig. 3 gezeigt weist das Gelenkarm-Segment 70 bzw. Drehgelenk 40 einen Arbeitsbereich A auf, welcher im Ausführungsbeispiel von der Neutralstellung N bis zur Endstellung E reicht. Von der Neutralstellung N und damit dem Beginn des Arbeitsbereichs A aus kann das Gelenkarm-Segment 70 in einem Schwenkbereich S über die obere vertikale Stellung V hinweg in die Parkposition P verschwenkt werden.

Die Neutralstellung N und damit der Beginn des Arbeitsbereichs A weist im Ausführungsbeispiel einen Winkel von ca. 15° zur oberen vertikalen Stellung V auf. Die Endstellung E weist einen Winkel von ca. 90° zur Vertikalen auf, d.h. sie verläuft im Wesentlichen horizontal. In der Parkposition P verläuft das Gelenkarm-Segment 70 mit einem Winkel von 180° zur oberen vertikalen Stellung V, d.h. im Wesentlichen vertikal nach unten. Der Schwenkbereich S von der Neutralposition N zur Parkposition P beträgt daher über 180°.

Im Arbeitsbereich A wird das Gelenkarm-Segment 70 durch eine Drehfederanordnung des Drehgelenks 40 gehalten, die das Gewicht des Spiegelgelenkarms 30 zumindest teilweise ausbalanciert. Dadurch, dass der Spiegelgelenkarm 30 sich im Arbeitsbereich A selbst hält, d.h. sein Eigengewicht zumindest teilweise kompensiert wird, ist ein im Wesentlichen ausbalanciertes Führen des Applikators 20 möglich.

Wird das Gelenkarm-Segment 70 aus dem Arbeitsbereich A über die Neutralposition N hinweg weiter in Richtung zur Parkposition P, bzw. zunächst zur oberen vertikalen Stellung V gekippt, so wird die Drehfederanordnung automatisch ausgekoppelt. Umgekehrt koppelt die Drehfederanordnung automatisch ein, wenn das Gelenkarm-Segment 70 aus der Parkposition P kommend die Neutralposition N erreicht.

Optional muss für das Verlassen der Neutralposition N in Richtung auf die Parkposition P und/oder von der Parkposition P kommend zum Erreichen der Neutralposition N ein Rastmoment überwunden werden.

Im Schwenkbereich S zwischen der Neutralposition N und der Parkposition P wirkt die Federanordnung dagegen nicht.

Optional muss zum Erreichen und/oder Verlassen der die Parkposition ein Rastmoment überwunden werden.

Das Ausklappen des Gelenkarm-Segments 70 aus der Parkposition P erfolgt entsprechend umgekehrt des Einklappens: optional Rastmoment überwinden, widerstandfrei bzw. widerstandsarm hochklappen, optional Rastmoment überwinden zum Eintreten in den Arbeitsbereich A, wobei die Drehfederanordnung automatisch wirksam wird und über den gesamten Arbeitsbereich A dem Gewicht des Gelenkarms 30 entgegenwirkt.

Die Bewegung des Gelenkarm-Segments 70 wird in der Endstellung E durch einen Anschlag gestoppt, so dass ein Überdrehen der Drehfederanordnung verhindert wird.

Die Drehfederanordnung ist daher nur in Arbeitsbereich A eingekoppelt, während das Einklappen und Ausklappen in und aus der Parkposition P widerstandsfrei bzw. widerstandsarm erfolgt.

Das optional vorgesehene zusätzliche Rastmoment erzeugt zusätzliche Sicherheit gegen unbeabsichtigtes Aus- / Einklappen.

Es ist kein Bedienelement notwendig, um die Drehfederanordnung ein- und auszukoppeln. Die Bedienung wird hierdurch einfacher und intuitiver, mögliche Bedienfehler werden minimiert.

Optional kann eine Bremse vorgesehen sein, welche über den gesamten Schwenkbereich des Gelenkarm-Segments 70 wirksam ist und daher insbesondere auch nach dem Verlassen des Arbeitsbereichs beim Einklappen des Gelenkarms 30 weiterhin wirksam bleibt und die Bewegung dämpft, so dass zu schnelle Bewegungen und mögliche Schäden am Gelenkarm 30 verhindert werden.

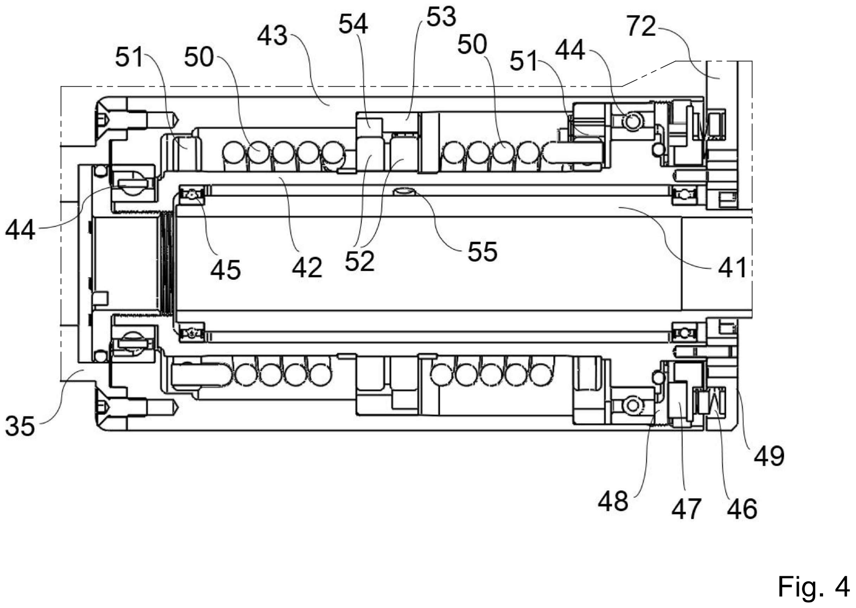

Eine mögliche konstruktive Ausgestaltung des Drehgelenks 40 wird anhand der Figuren 4 bis 10 beschrieben.

Das Drehgelenk 40 umfasst zwei relativ zueinander um eine horizontal ausgerichtete Drehachse drehbare Gelenkelemente 42 und 43, zwischen welchen im Arbeitsbereich A die Drehfederanordnung 50 wirkt. Die Drehfederanordnung 50 ist durch zwei gegenläufige Drehfedern gebildet, sodass ein Kraftausgleich bewirkt wird. Die Gelenkelemente 42, 43 sind über Wälzlager 44 drehbar aneinander gelagert. Anstelle eines oder sämtlicher Wälzlager 44 können Gleitlager eingesetzt werden.

Das Drehgelenk 40 stellt eine federnde Verbindung zwischen einer im Hinblick auf die Drehung um die Drehachse stationären Einheit auf Seiten der Laserquelle 15 und einer rotierenden Einheit auf Seiten des Gelenkarm-Segments 70 dar.

Im in Fig. 4 gezeigten Ausführungsbeispiel ist das zweite Gelenkelement 43 an der Basis 35 montiert und damit bezüglich einer Rotation um die Drehachse stationär, während das Gelenkarm-Segment 70 an dem ersten Gelenkelement 42 montiert ist und über dieses verschwenkbar ist.

Im in Fig. 4 gezeigten Ausführungsbeispiel handelt es sich bei dem ersten Gelenkelement 42 um eine Welle und bei dem zweiten Gelenkelement 43 um ein Gehäuse, welches die Welle umgibt. Eine umgekehrte Ausgestaltung des Drehgelenks wäre ebenfalls möglich. Entweder bildet daher wie im gezeigten Ausführungsbeispiel die stationäre Einheit ein Gehäuse und die rotierende Einheit eine darin liegende Welle, oder umgekehrt, wobei Welle und Gehäuse gegeneinander drehbar gelagert sind. Bei der Welle handelt es sich um eine Hohlwelle, in deren inneren der Laserstrahl durch das Drehgelenk 40 hindurchgeführt wird.

Im Arbeitsbereich A erfolgt eine Übertragung eines Drehmoments von dem ersten Gelenkelement 42 auf das zweite Gelenkelement 43 über die Drehfederanordnung 50. Die Federwirkung wird hierbei durch mindestens eine Drehfeder 50 erzielt. Hierbei kann eine Drehfeder, mehrere in gleicher Richtung wirkende Drehfedern, oder wie im in Fig. 4 gezeigten Ausführungsbeispiel zwei (oder mehr) paarweise gegenläufig gewickelte Drehfedern 50 eingesetzt werden. Durch die gegenläufige Wicklung der Drehfedern 50 wird ein Kraftausgleich zwischen den Federn erzielt.

Im in Fig. 4 gezeigten Ausführungsbeispiel sind die Drehfedern 50 auf einer Seite fest mit dem als Welle ausgebildeten ersten Gelenkelement 42 verbunden. Die andere Seite der Drehfedern 50 ist in einem gegenüber den beiden Gelenkelementen 42, 43, drehbaren Drehelement 52 gehalten. Im Ausführungsbeispiel handelt es sich bei dem Drehelement 52 um einen Ring, welcher das als Welle ausgebildete erste Gelenkelement 42 umgibt und zwischen Welle und Gehäuse angeordnet ist.

Das Drehelement 52 ist je nach Stellung des Gelenkarm-Segments 70 mit dem jeweils anderen Drehelement, d.h. im Ausführungsbeispiel mit dem als Gehäuse 43 ausgeführten zweiten Gelenkelement 43 im Eingriff.

In dem Schwenkbereich S zwischen der Parkposition P und der Neutralstellung N übertragen die Drehfedern 50 kein Moment zwischen den Gelenkelementen 42, 43, also zwischen der Welle und dem Gehäuse. Das Drehelement 52 ist in diesem Bereich nicht mit dem Gelenkelement 43 in Eingriff, d.h. im Ausführungsbeispiel mit dem Gehäuse. Es ist jedoch mit dem Gelenkelement 42 in Eingriff, mit dem auch das feste Ende der Drehfedern 50 verbunden ist. Die Drehfedern) 50 können in dieser Stellung optional bereits vorgespannt sein.

Im Arbeitsbereich A übertragen die Drehfedern 50 dagegen ein Drehmoment zwischen den Gelenkelementen 42, 43, indem das Drehelement 52 im Arbeitsbereich A mit dem als Gehäuse ausgebildeten zweiten Gelenkelement 43 in Eingriff steht.

Die Größe des von den Drehfedern 50 übertragenen Drehmoments hängt im Arbeitsbereich A von der Verdrehung zwischen Welle und Gehäuse und damit von der Stellung des Gelenkarm-Segments 70 ab. Dadurch wird das mit dem Anstellwinkel steigende Drehmoment, welches der Spiegelgelenkarm 30 durch sein Eigengewicht erzeugt, kompensiert.

Im in Fig. 4 gezeigten Ausführungsbeispiel erfolgt die Übertragung des Drehmoments daher von dem ersten Gelenkelement 42, hier der Welle, an welche die Drehfedern 50 befestigt ist, über die Drehfedern 50 auf das Drehelement 52. An dem Drehelement 52 ist ein Drehmomentübertragungselement 56 vorgesehen, welches im Arbeitsbereich A an einem Gegenelement 67 an dem zweiten Gelenkelement 43, hier dem Gehäuse, anliegt und das Drehmoment auf dieses überträgt. Beim Verlassen des Arbeitsbereichs A kommt das Drehmomentübertragungselement 56 dagegen selbsttätig außer Eingriff mit dem Gegenelement 67 und läuft im Schwenkbereich S zwischen der Neutralstellung N und der Parkposition P frei gegen das zweite Gelenkelement 43, hier das Gehäuse, so dass die Drehfedern 50 kein Drehmoment zwischen Gehäuse und Welle übertragen.

Weitere Details der Konstruktion werden im Folgenden beschrieben:
Das Drehgelenk 40 weist ein Strahlführungsrohr 41 auf, welches axial entlang der Drehachse durch das Drehgelenk 40 hindurchführt und in welchem der Laserstrahl koaxial zur Drehachse durch das Drehgelenk 40 hindurchgeführt wird. Das Strahlführungsrohr 41 bildet einen axial durch das Drehgelenk 40 hindurchgehenden Hohlraum, welcher auf seinen beiden axialen Enden mit Strahlführungsrohren der Basis 35 und des Gelenkarm-Segments 70 in Verbindung steht. In den Verbindungsbereichen des Strahlführungsrohrs 41 angeordnete Spiegel führen den Laserstrahl hierbei durch das Drehgelenk 40.

Wie in Fig. 2 dargestellt, weist das Gelenkarm-Segment 70 als Strahlführungsrohr ein Rohrsegment 71, in welchem der Laserstrahl verläuft, und einen sich parallel hierzu erstreckenden Stützarm 72 auf, welcher über das Drehgelenk 40 drehbar ist.. Hierbei ist der Stützarm 72 an dem ersten Gelenkelement 42 montiert, während das Rohrsegment 71 an dem Strahlführungsrohr 41 montiert ist, welches durch das erste Gelenkelement 42 hindurch verläuft und an diesem über Lager 45 drehbar gelagert ist. Hierdurch wird die Stützkraft, welche die Drehfederanordnung 50 erzeugt, erst am distalen Ende des Rohrsegments 71 in das Gelenkarm-Segment 70 eingeleitet.

Das Rohrsegment 71 könnte jedoch auch unmittelbar an dem ersten Gelenkelement 42 montiert sein, sodass auf das zusätzliche Strahlführungsrohr 41 verzichtet werden kann.

Wie bereits beschrieben bildet im Ausführungsbeispiel die feste, mit der Laserquelle 15 verbundene Einheit das Gehäuse. Die rotierende Einheit ist mit der im Gehäuse gelagerten Welle verbunden. Die Lagerung erfolgt im Ausführungsbeispiel mit vorgespannten Schrägkugellagern 44 in O-Anordnung.

Weiterhin werden zwei gegenläufig gewickelte Drehfedern 50 eingesetzt, um einen Kraftausgleich zu erreichen. Die Drehmomente der beiden Drehfedern 50 addieren sich hierbei, die Kräfte wirken gegenläufig auf die Welle bzw. das Gehäuse.

Jede Drehfeder 50 ist mit einer Seite fest mit dem als Welle ausgebildeten ersten Gelenkelement 42 verbunden. Die andere Seite der Drehfedern 50 ist in je einem Drehelement 52 aufgenommen, die auf der Welle drehbar montiert sind. Die Drehelemente 52 sind als Ringe ausgestaltet, welche auf die Welle aufgeschoben und axial mit Sicherungsringen 62 gesichert werden.

Im Ausführungsbeispiel stehen die beiden Drehfedern 50 mit ihren äußeren Enden mit der Welle und mit ihren inneren Enden mit den Drehelementen 52 in Verbindung, welche nebeneinander auf der Welle angeordnet sind.

In jedem der beiden Drehelemente 52 steckt ein Zylinderstift 58, dessen äußeres Ende das Drehübertragungselement 56 bildet. Zwischen dem Arbeitsbereich A und der Parkposition P läuft das Drehübertragungselement 56 frei im Gehäuse, insbesondere in einer Nut 54 am Gehäuse. Beim Eintritt in den Arbeitsbereich A kommt das Drehübertragungselement 56 in der Neutralstellung N mit einem Anschlag 67 des Gehäuses in Anlage, welcher damit ein Gegenelement bildet, auf welchem sich das Drehübertragungselement 56 im Arbeitsbereich A abstützt. Bei dem Anschlag handelt es sich um das Ende 67 der Nut 54. Der Drehmomentfluss geht von der Drehfeder 50 durch das Drehelement 52 und das Drehübertragungselement 56 auf den Anschlag 67 im Gehäuse. Dadurch wird ein Drehmoment zwischen fester und rotierender Einheit übertragen, das so abgestimmt ist, dass die Gewichtskraft des Spiegelgelenkarms 30 kompensiert wird .

Das andere Ende der Nut 54 kommt bei einer Drehbewegung der Welle über die Parkposition P hinaus mit dem Drehübertragungselement 56 in Anschlag und verhindert in diese Drehrichtung ein Überdrehen. Am anderen Ende der Nut 54 ist ein axialer Durchbruch 68 vorgesehen, welcher es erlaubt, das Drehelement 52 mit dem Drehübertragungselement 56 in das Gehäuse einzuschieben.

Zusätzlich ist eine in axialer Richtung vorgespannte Bremse verbaut, bestehend aus einer Stahlscheibe 48 sowie einer Aluminiumscheibe mit aufgeklebten Bremsbelägen 47, die durch eine Wellenfeder 46 mit einer definierten Kraft gegeneinander gedrückt werden. Die Wellenfeder 46 ist hierbei in einem mit der Welle verbundenen Gehäuseelement 49 gelagert. Die Stahlscheibe 48 ist auf das axiale Ende des Gehäuses aufgeschraubt.

Alternativ dazu kann eine Bremse durch einen O-Ring realisiert sein, welcher an seinem Innenumfang in der Nut zwischen den radialen Außenflächen der Drehelemente 52, und an seinem Außenumfang an der radialen Innenfläche des Gehäuse 43 aufliegt. Der Übersichtlichkeit halber ist eine solche Ausführung nicht in den Figuren dargestellt.

Der Zylinderstift 58 geht im Ausführungsbeispiel durch das Drehelement 52 radial hindurch, wobei das innere Ende ein zweites Drehmomentübertragungselement 57 bildet, welches sich im Arbeitsbereich frei in einer Nut 55 in der Welle bewegt. Die beiden Enden der Nut 55 begrenzen den Drehwinkelbereich, in welchem das Drehelement 52 gegenüber der Welle drehbar ist.

Das eine Ende der Nut 55 bildet ein Gegenelement 66, welches die Drehbewegung des Drehelements 52 gegenüber der Welle in einer Richtung gegen die Kraft der Drehfedern 50 und damit den Arbeitsbereich A begrenzt, und mit welchem das zweite Drehmomentübertragungselement 57 in der Endstellung E in Anschlag kommt. In dieser Endstellung E stützt sich die Welle daher über das Gegenelement 66, das zweite Drehmomentübertragungselement 57, das Drehelement 52, das erste Drehmomentübertragungselement 56 und das Gegenelement 67 ohne eine Zwischenschaltung der Drehfedern 50 auf dem Gehäuse ab. Hierdurch wird der Arbeitsbereich A begrenzt und ein Überdrehen der Drehfedern 50 vermieden.

Das andere Ende der Nut 55 bildet ein weiteres Gegenelement 65, auf welchem sich das zweite Drehmomentübertragungselement 57 im Schwenkbereich S zwischen dem Arbeitsbereich A und der Parkposition P abstützt und daher den Drehbereich des Drehelementes 52 in Richtung auf eine kraftlose Position der Drehfedern 50 begrenzt. Hierdurch liegt das Drehelement 52 im Schwenkbereich S mit einer durch die Drehfedern 50 erzeugten Vorspannung an der Welle an. Kommt das Drehelement 52 daher bei einer Bewegung von der Parkposition P in Richtung auf den Arbeitsbereich A in der Neutralstellung N über das Drehmomentübertragungselement 56 und das Gegenelement 67 mit dem Gehäuse in Anlage, weist das Drehelement 52 bereits eine Vorspannung auf, welche einer weiteren Drehbewegung in Richtung auf die Endstellung E entgegenwirkt. Bevorzugt ist die Vorspannung hierbei so groß gewählt, dass das durch sie erzeugte Drehmoment jenes Drehmoment, welches der Spiegelgelenkarm 30 durch sein Eigengewicht in der Neutralstellung N erzeugt, ausgleicht.

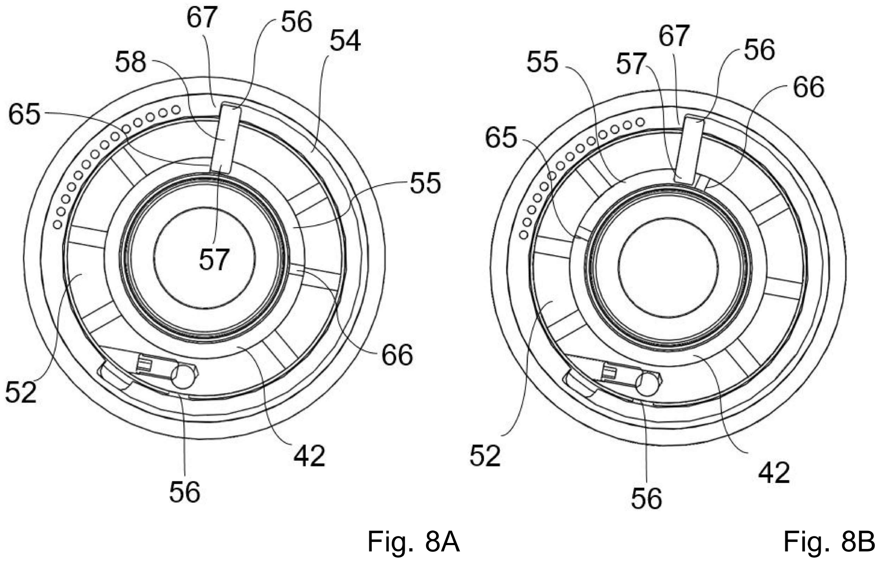

Fig. 8A zeigt das Drehgelenk 40 in der Neutralstellung N. Hierbei kommt das Drehmomentübertragungselement 56 des Drehelements 52 und das Gegenelement 67 des Gehäuses in Anlage. Weiterhin befindet sich das zweite Drehmomentübertragungselement 57 noch in Anlage mit dem Gegenelement 65 der Welle.

Wird die Welle weiter in Richtung auf die Endstellung E gedreht, so kommt das zweite Drehmomentübertragungselement 57 außer Anlage mit dem Gegenelement 65 der Welle und bewegt sich frei in der Nut 55 der Welle, während das Drehmomentübertragungselement 56 des Drehelements 52 mit dem Gegenelement 67 des Gehäuses in Anlage bleibt, so dass Welle und Gehäuse über die Drehfedern 50 in Wirkverbindung stehen.

Fig. 8B zeigt das Drehgelenk 40 in der Endstellung E, in welcher das zweite Drehmomentübertragungselement 57 in Anlage mit dem Gegenelement 66 der Welle kommt, so dass sich das Gegenelement 67 des Gehäuses und das Gegenelement 66 der Welle über das Drehelement 52 aufeinander abstützen. Eine weitere Drehung der Welle wird daher verhindert.

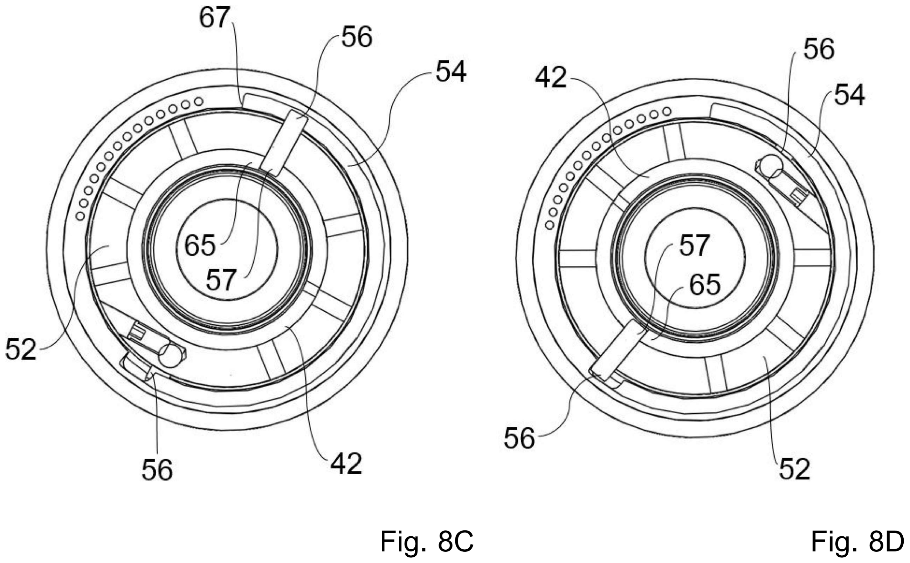

Wird die Welle dagegen aus der in Fig. 8A gezeigten Neutralstellung N in Richtung auf die Parkposition P gedreht, so kommt das Drehmomentübertragungselement 56 wie in Fig. 8C gezeigt außer Anlage mit dem Gegenelement 67 des Gehäuses und bewegt sich frei in der Nut 54 des Gehäuses. Hierdurch ist ein widerstandsfreies bzw. widerstandsarmes Einschwenken des Spiegelgelenkarm 30 in die Parkposition P möglich. Im Schwenkbereich S ist das zweite Drehmomentübertragungselement 57 des Drehelements 52 mit dem Gegenelement 65 der Welle in Anlage, um die Vorspannung aufrecht zu erhalten.

Fig. 8D zeigt die Parkposition P, in welcher das Drehmomentübertragungselement 56 mit einem am anderen Ende der Nut 54 des Gehäuses vorgesehenen Gegenelement in Anschlag kommt, welches eine weitere Drehbewegung über die Parkposition P hinaus verhindert.

Im Ausführungsbeispiel werden das Drehmomentübertragungselement 56 und das zweite Drehmomentübertragungselement 57 durch die beiden Enden eines Stiftes 58 gebildet, welcher durch das Drehelement 52 hindurch geht. Die beiden Drehmomentübertragungselemente 56, 57 könnten jedoch auch durch separate Stifte, welche in separaten Bohrungen des Drehelements 52 angeordnet sind, oder durch an dem Drehelement 52 in anderer Weise angeformte Elemente gebildet werden.

Weiterhin stehen die Drehmomentübertragungselemente 56, 57 im Ausführungsbeispiel von dem Drehelement 52 in radialer Richtung nach außen und innen ab und verlaufen in Nuten, deren Enden die Gegenelemente 66, 67 bilden. In alternativen Ausgestaltungen könnte auf Nuten jedoch verzichtet werden und die Gegenelemente 66, 67 könnten durch Elemente gebildet werden, welche von der Welle nach außen bzw. von dem Gehäuse nach innen vorspringen. Weiterhin könnten die Drehmomentübertragungselemente 56, 57 auch in axialer Richtung seitlich von dem Drehelement 52 abstehen und mit entsprechenden Gegenelementen auf Welle und Gehäuse zusammenwirken.

Ebenso wäre es denkbar, die oben beschriebenen Möglichkeiten für die Drehmomentübertragungselemente und die Gegenelemente im Hinblick auf ihre Anordnung an dem Drehelement und den beiden Gelenkelementen umzukehren und beispielsweise an der Welle und am Gehäuse Stifte vorzusehen, welche in Nuten im Drehelement verlaufen und/oder mit Gegenelementen am Drehelement zusammenwirken.

Im Ausführungsbeispiel erfolgt die Befestigung der Drehfedern 50 an der Welle über je einen Ring 51, in welchem das äußere Ende der Drehfeder 50 gehalten ist und welcher auf die Welle 42 aufgeschoben und über radial durch den Ring 51 hindurchgehende Schrauben 61 mit der Welle verschraubt oder verspannt wird.

Hierbei wird für die Ringe 51 ein identisches Bauteil wie für die Drehelemente 52 genutzt, in welchen das jeweils innere Ende der Drehfedern 50 befestigt ist. Die Befestigung der Drehfedern 50 erfolgt dadurch, dass ein axiales Ende der Drehfedern 50 durch eine axiale Öffnung in dem Ring 51, 52 hindurchgeht und durch eine Spannschraube 64 gesichert wird.

Im Ausführungsbeispiel sind die Nuten 54 bzw. Gegenelemente 67 des Gehäuses in einem Ringelement 53 vorgesehen, welches an der Innenwand des Gehäuses befestigt ist. Das Gegenelement weist hierbei die Nuten 54 bzw. Gegenelemente 67 für beide Drehübertragungselemente 56 der beiden Drehelemente 52 auf.

Alternativ könnten die Drehfedern 50 jedoch auch in beliebiger anderer Art und Weise mit der Welle befestigt werden. Ebenso könnten die Gegenelemente 67 in anderer Art am Gehäuse angebracht werden.

Weiterhin alternativ könnten die Drehfedern 50 auch mit dem Gehäuse fest verbunden und über die Drehelemente mit der Welle je nach Drehstellung gekoppelt werden.

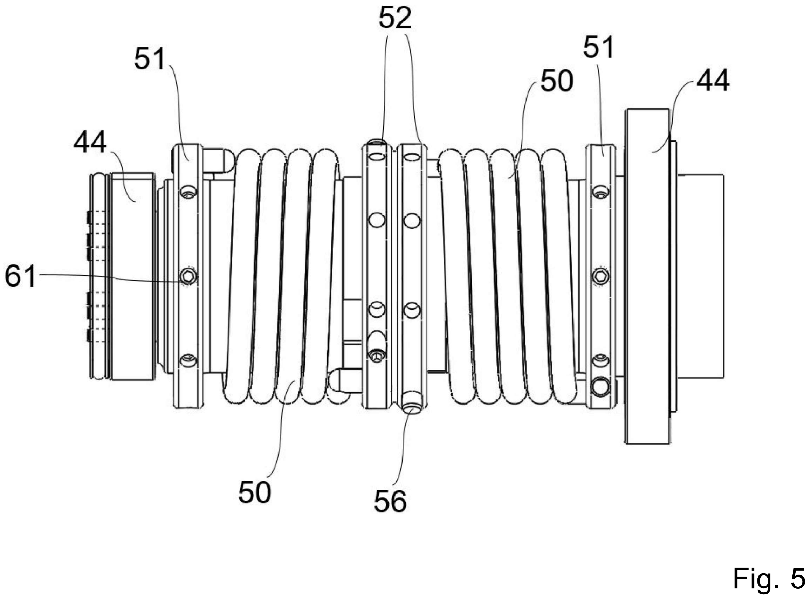

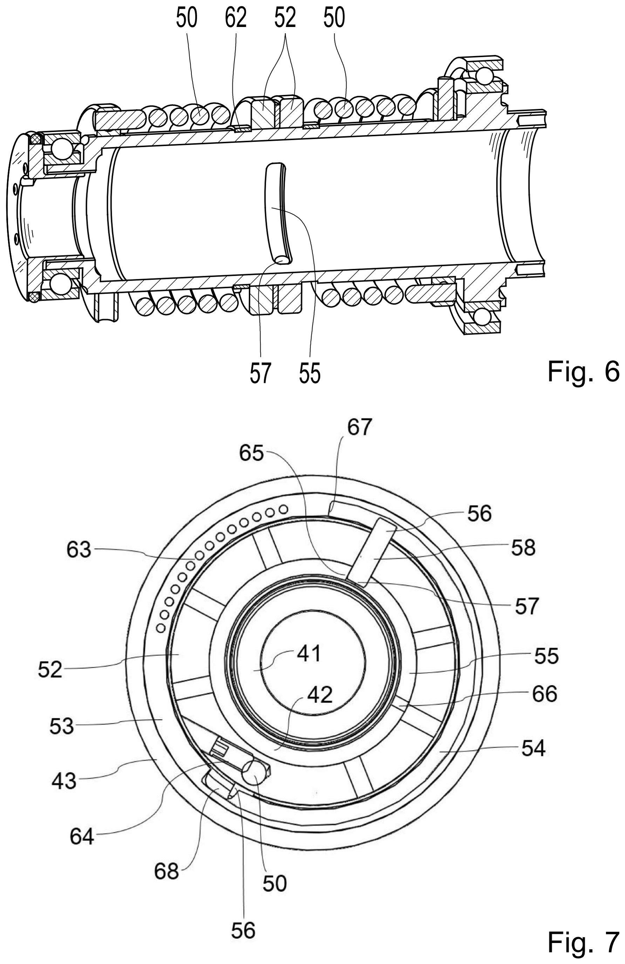

Wie in Fig. 5 und Fig. 6 ersichtlich sind die beiden Drehfedern 50 nicht nur gegenläufig gewickelt, sondern auch auf gegenüberliegenden Seiten der Drehachse mit dem jeweiligen Drehelement 52 verbunden. Die Drehmomentübertragungselemente 56, 57 bzw. der Stift 58 sowie die entsprechenden Nuten 54, 55 sind auf gegenüberliegenden Seiten bezüglich der Drehachse angeordnet.

Fig. 7 zeigt eine Schnittansicht durch das Drehgelenk 40 senkrecht zur Drehachse. Aus dieser Darstellung geht deutlich hervor, dass die beiden Drehmomentübertragungselemente 56, 57 auf gegenüberliegenden Seiten angeordnet sind, sodass ein Kraftausgleich der beiden Drehfedern 50 bewirkt wird.

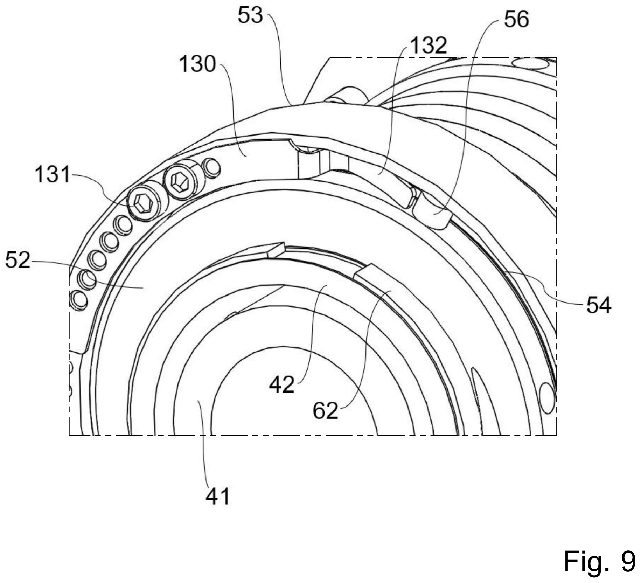

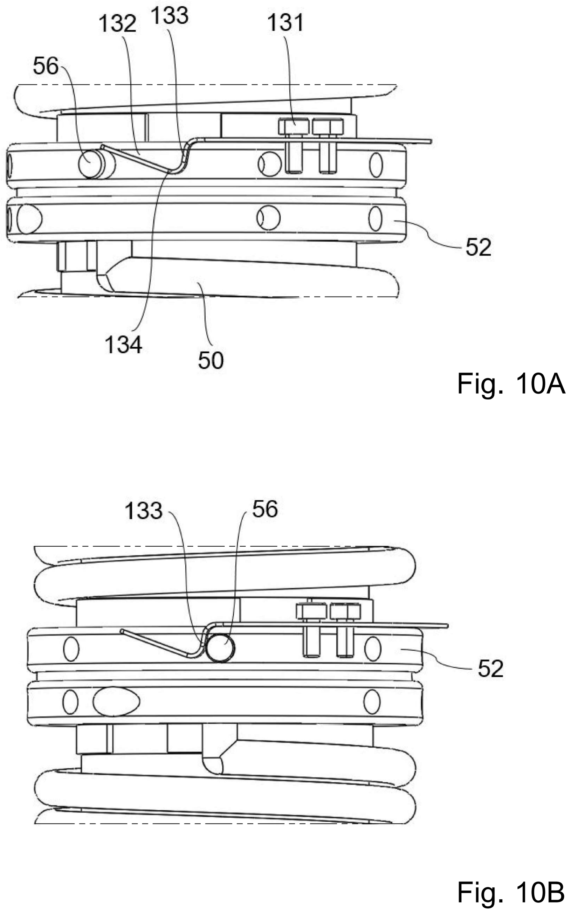

Wie in Fig. 9 und 10 gezeigt sind in der Neutralstellung N sowie in der Parkposition P Rastelemente 130 in Form von gebogenen Federblechen verbaut. Dadurch rastet der Spiegelgelenkarm 30 in der Neutralstellung N sowie in der Parkposition P ein. Um den Arm beim Einklappen aus der oberen Neutralstellung N in Richtung auf die Parkposition P bzw. aus der Parkposition P nach oben auszuklappen, muss daher ein Rastmoment überwunden werden.

Fig. 9, Fig. 10A und Fig. 10B zeigen das Rastelement 130 für die Neutralstellung N. Das Rastelement 130 ist wie in Fig. 9 gezeigt seitlich am Ringelement 53 über Schrauben 131 angebracht, welche in Bohrungen 63 am Ringelement 53 eingreifen. Das Rastelement 130 wirkt mit dem Drehmomentübertragungselement 56 zusammen. Hierfür ragt eine Rastnase 134 des Rastelements 130 in die Nut 54 am Ringelement 53 hinein. Wie in Fig. 10A gezeigt muss das Drehmomentübertragungselement 56 bei einer Bewegung in die Neutralstellung N die Anlauffläche 132 der Rastnase 134 zur Seite drücken, bis sie an der Rastnase 134 vorbei gelangt ist. In die Richtung aus der Neutralstellung N in die Parkposition P muss das Drehmomentübertragungselement 56 wie in Fig. 10B gezeigt die Rastkante 133 überwinden. Die Rastkraft bei einer Bewegung aus der Neutralstellung N in die Parkposition P ist daher größer als in die umgekehrte Richtung.

Das Rastelement für die Parkposition P ist dagegen, in den Figuren nicht zu erkennen, an einem weiteren Ringelement des Gehäuses angebracht und wirkt mit einem Stift zusammen, welcher an einem der Ringelemente 51 angebracht ist. Eine umgekehrte Anordnung wäre ebenfalls denkbar.

## Patentansprüche

1. Drehgelenk (40) für einen Spiegelgelenkarm (30) eines Lasersystem (10) zur Übertragung von Laserenergie von einer Laserquelle (15) zu einem Applikator (20), mit einem ersten Gelenkelement (42) und einem zweiten Gelenkelement (43), welche um eine Drehachse drehbar miteinander gekoppelt sind, wobei das erste Gelenkelement (42) und das zweite Gelenkelement (43) innerhalb eines Arbeitsbereichs (A) gegeneinander verschwenkbar sind, wobei das Drehgelenk (40) eine Drehfederanordnung (50) umfasst, die im Arbeitsbereich (A) mit dem ersten Gelenkelement (42) und dem zweiten Gelenkelement (43) in Eingriff steht und ein Drehmoment zwischen diesen überträgt, um im Arbeitsbereich (A) zumindest einen Teil des Eigengewichts eines mit einem der Gelenkelemente (42, 43) verbundenen Elements (30) zu kompensieren, wobei das Drehgelenk (40) von dem Arbeitsbereich (A) in eine Parkposition (P) und zurück verschwenkbar ist,
wobei das Drehgelenk (40) dazu eingerichtet ist, die Drehfederanordnung (50) beim Verlassen des Arbeitsbereiches (A) in Richtung auf die Parkposition (P) mit mindestens einem der Gelenkelemente (42, 43) selbsttätig außer Eingriff zu bringen, so dass die Drehfederanordnung (50) einer Schwenkbewegung in die Parkposition (P) kein Drehmoment entgegensetzt.

2. Drehgelenk (40) nach Anspruch 1, mit einem Anschlag (66), welcher dazu eingerichtet ist den Arbeitsbereich (A) auf der der Parkposition (P) abgewandten Seite zu begrenzen und so ein Überdrehen der Drehfederanordnung (50) zu verhindern.

3. Drehgelenk (40) nach Anspruch 1 oder 2, mit mindestens einem Rastelement (130), welches zur Erzeugung eines Rastmoments eingerichtet ist, das beim Verlassen des Arbeitsbereiches (A) und/oder beim Verlassen der Parkposition (P) überwunden werden muss, wobei das Rastelement bevorzugt durch zumindest ein Federblech (130) gebildet ist, welches weiter bevorzugt mit einem Stiftelement (56) zusammenwirkt.

4. Drehgelenk (40) nach einem der vorangegangenen Ansprüche, mit einer Bremsanordnung (46 - 48) zum Abbremsen einer Verdrehung zwischen dem ersten und dem zweiten Gelenkelement (42, 43), wobei die Bremsanordnung (46 - 48) zumindest in einem Schwenkbereich (S) zwischen dem Arbeitsbereich (A) und der Parkposition (P) wirksam oder betätigbar ist.

5. Drehgelenk (40) nach einem der vorangegangenen Ansprüche, wobei die Drehfederanordnung mindestens zwei Drehfedern (50) aufweist, welche bevorzugt gegenüberliegend angeordnet sind und/oder bevorzugt unabhängig voneinander mit mindestens einem der Gelenkelemente (42, 43) in Eingriff und außer Eingriff kommen.

6. Drehgelenk (40) nach einem der vorangegangenen Ansprüche, wobei das Drehgelenk (40) derart eingerichtet ist, dass die Drehfederanordnung (50) in einem Schwenkbereich (S) zwischen der Parkposition (P) und dem Arbeitsbereich (A) außer Eingriff mit dem mindestens einen Gelenkelement (42, 43) bleibt und/oder wobei die Drehfederanordnung (50) bei einer Bewegung aus der Parkposition (P) zum Arbeitsbereich (A) beim Eintritt in den Arbeitsbereich (A) selbsttätig mit mindestens einem der Gelenkelemente (42, 43) in Eingriff kommt, und/oder wobei die Drehfederanordnung (50) im Schwenkbereich (S) zwischen der Parkposition (P) und dem Arbeitsbereich (A) eine Vorspannung aufweist, welche beim Eintritt in den Arbeitsbereich (A) einer weiteren Drehbewegung entgegenwirkt.

7. Drehgelenk (40) nach einem der vorangegangenen Ansprüche, wobei die Drehfederanordnung (50) mindestens ein Drehmomentübertragungselement (56) aufweist, welches dazu eingerichtet ist im Arbeitsbereich (A) in Eingriff mit mindestens einem Gegenelement (67) zu kommen, um ein Drehmoment auf das Gegenelement (67) zu übertragen, wobei das Drehmomentübertragungselement (56) und das Gegenelement (67) dazu eingerichtet sind, beim Verlassen des Arbeitsbereiches (A) selbsttätig außer Eingriff zu kommen, wobei es sich bei dem Drehmomentübertragungselement (56) und/oder dem Gegenelement (67) bevorzugt um einen Anschlag handelt, wobei weiter bevorzugt das Drehmomentübertragungselement (56) und/oder das Gegenelement (67) dazu eingerichtet sind, bei einer Schwenkbewegung von der Parkposition (P) in Richtung auf den Arbeitsbereich (A) beim Eintritt in den Arbeitsbereich (A) miteinander in Anlage kommen, so dass die Drehfederanordnung (50) einer weiteren Schwenkbewegung ein Drehmoment entgegensetzt, und/oder wobei das Drehmomentübertragungselement (56) und das Gegenelement (67) bei einer Schwenkbewegung in die Gegenrichtung bei Verlassen des Arbeitsbereichs (A) außer Kontakt kommen, wobei bevorzugt das Drehmomentübertragungselement (56) im Arbeitsbereich (A) in Eingriff mit dem Gegenelement (67) und in einem Schwenkbereich (S) zwischen dem Ende des Arbeitsbereiches (A) und der Parkposition (P) außer Eingriff mit dem Gegenelement (67) steht.

8. Drehgelenk (40) nach Anspruch 7, wobei es sich bei dem Drehmomentübertragungselement (56) oder bei dem Gegenelement (67) um ein Stiftelement (58) und bei dem jeweils anderen Element um das Ende einer Nut (54) handelt, in welcher sich das Stiftelement (58) bewegt.

9. Drehgelenk (40) nach einem der Ansprüche 7 oder 8, wobei die Drehfederanordnung mindestens eine Drehfeder (50) umfasst, deren erstes Ende mit dem ersten Gelenkelement (42) gekoppelt ist und deren zweites Ende mit einem Drehelement (52) gekoppelt ist, welches gegenüber dem ersten und dem zweiten Gelenkelement (42, 43) drehbar angeordnet ist, wobei das Drehmomentübertragungselement (56) an dem Drehelement (52) angeordnet ist und mit dem Gegenelement (67) zusammenwirkt, welches mit dem zweiten Gelenkelement (43) gekoppelt ist, wobei es sich bei dem Drehelement (52) bevorzugt um ein Ringelement handelt, welches drehbar um das erste oder zweite Gelenkelement (42, 43) und/oder drehbar zwischen dem ersten und dem zweiten Gelenkelement (42, 43) angeordnet ist.

10. Drehgelenk (40) nach Anspruch 9, wobei das Drehelement (52) an mindestens einem Ende des Arbeitsbereiches (A) in Eingriff mit dem ersten Gelenkelement (42) kommt, so dass eine weitere Relativdrehung zwischen dem Drehelement (52) und dem ersten Gelenkelement (42) verhindert wird, wobei an dem Drehelement (52) bevorzugt ein zweites Drehmomentübertragungselement (57) angeordnet ist, welches an dem mindestens einem Ende des Arbeitsbereiches (A) mit mindestens einem zweiten Gegenelement (66), welches an dem ersten Gelenkelement (42) angeordnet ist, in Eingriff kommt, wobei es sich bei dem mindestens einen zweiten Gegenelement (66) und/oder zweiten Drehmomentübertragungselement (57) bevorzugt um einen Anschlag handelt, und/oder wobei bevorzugt das zweite Drehmomentübertragungselement (57) oder das zweite Gegenelement (66) durch ein Stiftelement (58) und das jeweils andere Element durch das Ende einer Nut (55), in welcher sich das Stiftelement (58) bewegt, gebildet wird.

11. Drehgelenk (40) nach einem der vorangegangenen Ansprüche, mit einem Strahlführungsbereich, welcher entlang der Drehachse durch das Drehgelenk (40) hindurchführt, so dass ein Laserstrahl koaxial zur Drehachse durch das Drehgelenk (40) hindurchführbar ist, insbesondere in Form eines Strahlführungsrohrs (41), welches durch das Drehgelenk (40) hindurchführt und die Drehachse umgibt.

12. Drehgelenk (40) nach einem der vorangegangenen Ansprüche, wobei es sich bei den beiden Gelenkelementen um eine Welle (42) und ein die Welle umgebendes Gehäuse (43) handelt, wobei die Welle (42) drehbar im Gehäuse (43) gelagert ist, wobei bevorzugt eine Drehfeder (50) der Drehfederanordnung die Welle (42) umgibt und im Bereich zwischen der Welle (42) und dem Gehäuse (43) angeordnet ist, und/oder wobei es sich bei der Welle (42) bevorzugt um eine Hohlwelle handelt, innerhalb welcher sich ein Strahlführungsbereich axial durch das Drehgelenk (40) hindurch erstreckt, wobei weiter bevorzugt innerhalb der Welle (42) ein Strahlführungsrohr (41) drehbar gelagert ist, welches koaxial durch das Drehgelenk (40) hindurch führt, und/oder wobei bevorzugt an dem Drehgelenk (40) ein Stützarm (72) angeordnet oder anordenbar ist, welcher dazu eingerichtet ist ein Strahlführungsrohr (41) des Gelenkarm-Segments (70) zu stützen.

13. Spiegelgelenkarm (30) für ein Lasersystem (10) zur Übertragung von Laserenergie von einer Laserquelle (15) zu einem Applikator (20), mit einer Basis (35), einem Gelenkarm-Segment (70) und einem Drehgelenk (40) nach einem der vorangegangenen Ansprüche, wobei eines der Gelenkelemente (42, 43) des Drehgelenks (40) mit dem Gelenkarm-Segment (70) verbunden oder verbindbar ist, und das andere der Gelenkelemente (42, 43) des Drehgelenks (40) mit der Basis (35) verbunden oder verbindbar ist, sodass durch Verschwenken der beiden Gelenkelemente (42, 43) eine Arbeitsposition des Gelenkarm-Segments (70) veränderbar ist, wobei die Drehfederanordnung (50) des Drehgelenks (40) dazu eingerichtet ist zumindest einen Teil des Eigengewichts des Gelenkarm-Segments (70) zu kompensieren.

14. Spiegelgelenkarm (30) nach Anspruch 13, wobei die Drehachse des Drehgelenks (40) horizontal angeordnet ist, und/oder wobei der Spiegelgelenkarm (30) eine Mehrzahl von gegeneinander drehbaren Gelenkarm-Segmenten (70, 90, 120) aufweist, wobei das Drehgelenk (40) mit der Federanordnung (50) bevorzugt zwischen einer um eine vertikal ausgerichteten Achse drehbaren Basis (35) und einem ersten Gelenkarm-Segment (70) des Spiegelgelenkarms (30) angeordnet ist, und/oder wobei sich der Arbeitsbereich (A) und zumindest ein Teil des Schwenkbereichs (S) zwischen dem Arbeitsbereich (A) und der Parkposition (P) bezüglich der Vertikalen gegenüberliegen, sodass das Gelenkarm-Segment (70) nach dem Verlassen des Arbeitsbereichs (A) auf dem Weg zur Parkposition (P) durch eine obere vertikale Stellung (V) schwenkbar ist, wobei ein Beginn (N) des Arbeitsbereichs (A) bevorzugt einen Winkelabstand zur einer oberen vertikalen Stellung (V) von mindestens 5°, insbesondere von mindestens 10° und/oder von höchstens 25°, bevorzugt höchstens 20° aufweist, und/oder wobei das Gelenkarm-Segment (70) in der Parkposition (P) in einem Winkel von höchstens 10° zu einer unteren vertikalen Stellung steht, und/oder wobei der Arbeitsbereich (A) eine Größe von weniger als 90° aufweist und/oder wobei der Schwenkweg von dem Arbeitsbereich (A) zur Parkposition (P) eine Größe von mindestens 180° aufweist.

15. Lasersystem (10), insbesondere medizinisches Lasersystem, mit einer Laserquelle (15) und/oder einem Applikator (20) und einem Spiegelgelenkarm (30) nach einem der Ansprüche 13 oder 14.
